# EUROPÄISCHE PATENTSCHRIFT

(11) **EP 0 671 377 B1**
(45) Veröffentlichungstag und Bekanntmachung des Hinweises auf die Patenterteilung: **20.05.1998**
(21) Anmeldenummer: 95102760.6
(22) Anmeldetag: 27.02.1995
(51) Int. Cl.: C07C 37/84, C07C 39/16

(54) **Verfahren zur kontinuierlichen Herstellung von hochreinem Bisphenol-A**
Method of continuous production of highly pure bisphenole-A
Procédé de production continuelle de bisphénol-A à haute pureté

(30) Priorität: 10.03.1994 DE 4408008
(43) Veröffentlichungstag der Anmeldung: 13.09.1995
(73) Patentinhaber: BAYER AG, 51368 Leverkusen (DE)
(72) Erfinder: Berg, Klaus, Dr., D-47798 Krefeld (DE); Wulff, Claus, Dr., D-47800 Krefeld (DE); Malamet, Georg, Dr., D-47800 Krefeld (DE); Eitel, Alfred, Dr., D-41539 Dormagen (DE); Meurer, Kurt Peter, Dr., B-20000 Antwerpen (BE); van Osselaer, Tony, B-9111 St. Niklaas, Belsele (BE); Hinz, Jürgen, Dr., B-2930 Brasschaat (BE)

(56) Entgegenhaltungen:
- DE-A- 4 213 872

## Beschreibung

Die Erfindung betrifft ein Verfahren zur Herstellung von hochreinem Bisphenol-A (BPA), das weniger als 0,2 Gew.-% Verunreinigungen enthalt. So hergestelltes, thermo- und farbstabiles Bisphenol-A berwirkt eine verbesserte Thermofarbstabilität in daraus hergestellten Polymeren wie z.B. Polycarbonat.

Die Herstellung von hochreinem Bisphenol-A ist bekannt (z.B. DE-A 42 13 872). Hier wird beschrieben wie hergestelltes Bisphenol-A bis auf einen Reinheitsgrad von 99,91 Gew.-% gebracht werden kann. Jedoch kann die Farbzahl nach Hazen noch weiter verbessert werden.

Es wurde nun gefunden, daß man Bisphenol-A großtechnisch mit hohem Reinheitsgrad und guter Hazen-Farbzahl herstellen kann. Die hohe Reinheit des Endproduktes p,p-Bisphenol-A wird verfahrenstechnisch fast ausschließlich durch die BPA-/Phenol-Addukt-Kristallisation bestimmt.

Gegenstand der Erfindung ist daher ein Verfahren zur Gewinnung von Bisphenol-A mit einem Reinheitsgrad von 99,75 bis >99,94 Gew.-% und einer Farbzahl nach Hazen von 5 bis 10 aus der Reaktionslösung, die bei der Herstellung von Bisphenol-A aus Aceton und Phenol in Gegenwart von gegebenenfalls mit Mercaptoaminen und/oder Thiazolidinen und/oder Thiocarbonsäuren modifizierten sulfonsauren Ionenaustauschern erhalten wird, dadurch gekennzeichnet, daß
1. die Reaktionslösung bei einer Temperatur von 65 bis 75°C auf einen Gehalt an 25 bis 35 Gew.-% p,p-Bisphenol-A in Phenol eingestellt wird und
2. anschließend bei dieser Temperatur in eine n-stufige, seriengeschaltete Kristallerkaskade mit einer Anzahl an Kristallisationsreaktoren n > 1 eingespeist wird und
3. mit einer Verweilzeit des Gemisches pro Kristallisationsreaktor von mindestens drei Stunden und einer Umpumpleistung von mindestens 500 m³/h im jeweiligen Kristallisationsreaktor umgepumpt wird und
4. über die gesamte Kristallisationskaskade ein Temperaturgefalle von 70°C im ersten Kristallisationsreaktor (n = 1) auf 40°C im letzten Kristallisationsreaktor n eingestellt wird und
5. p,p-Bisphenol-A-Addukt-Kristallisate mit einem Gehalt an p,p-BPA von ca. 60 % und Phenol von ca. 40 % in den Kristallreaktoren gefüllt, dann abfriltriert, Phenol entfernt und p,p-Bisphenol in üblicher Weise gewonnen wird.

Die im erfindungsgemäßen Verfahren verwendbaren modifizierten sulfonsauren Ionenaustauscher als Katalysator sind bekannt (z.B. DE-A 37 27 641, entsprechend US-A 49 12 263).

Die Reaktionslösung wird durch Einstellen (z.B. durch Abwiegen, Dosierventile u.a.) auf einen Gehalt von 25 bis 35 Gew.-% Bisphenol-A in Phenol gebracht, was z.B. durch Gaschromatographie ermittelt werden kann.

Die aus der Umsetzung von Phenol und Aceton erhaltene phenolische Bisphenol-A-Lösung wird in eine mehrstufige Kristallisationskaskade für die Kristallisation des Bisphenol-A/Phenol 1:1 Adduktes eingeleitet. Die erfindungsgemäß verwendete Kaskade hat zwei oder drei Kristaller (n = 2 oder 3), kann aber auch mehr haben (n >3). Das aus dieser Kaskade abgezogene Produkt wird filtriert und die abfiltrierten Bisphenol-A/Phenol-Mischkristalle (1:1 Addukt) werden durch Desorption in Phenol und Bisphenol-A aufgetrennt, welches z.B. in Form von Kristallschuppenplättchen anfällt.

Die Kristallerkaskade hat mindestens zwei Kristallisatoren (Kristaller n, n > 1). Vorteilhafterweise werden 3 bis 6 Kristallisatoren in Serie geschaltet.

Die Temperatur wird so geregelt, daß über die gesamte Kristallisationskaskade ein Temperaturbereich von 70 (Reaktoreingang Reaktor n = 1) bis 40°C durchlaufen wird. Im ersten Kristaller (n = 1) wird bei höchstens 70°C gearbeitet und im letzten Kristaller n wird eine Temperatur von 40°C erreicht.

Anschließend wird Bisphenol-A in üblicher Weise abgetrennt. Es hat einen Reinheitsgrad von mindestens 99,75 Gew.-% Bisphenol-A und eine Farbzahl nach Hazen von 5 bis 10.

Das besondere an dieser speziellen Addukt-Kristallisation ist, daß hochreines BPA (>99,94 %) durch eine Verbesserung einer in-prozeß-Kristallisation gewonnen werden kann, ohne einen dem BPA-Verfahren nachgeschalteten Prozeß-Kristallisationsschritt, bei dem eine BPA-Schmelze in einem Lösemittel (wie Phenol oder Toluol oder Methylenchlorid oder Aceton) umkristallisiert wird.

Die BPA-/Phenol-Addukt-Kristallisation mit nachgeschalteter Filtration und Desorption ist wirtschaftlicher als ein separater Umkristallisationsschritt.

### Beispiele

### Beispiel 1

22 m³/h einer 28 %igen phenolischen Bisphenol-A-Lösung wurde mit einer Temperatur von 70°C in einen Kristaller eingespeist und im Kristaller auf 54°C abgekühlt. Der entstehende, phenolische BPA-/Phenol-Mischkristallbrei wurde kontinuierlich mit 1200 m³/h umgewälzt. Aus dem Kristaller wird der Mischkristallbrei, der eine Temperatur von 54°C besitzt, ausgespeist und in einen zweiten Kristaller eingespeist (22 m³/h). Im 2. Kristaller wird der Mischkristallbrei auf 41°C weiter heruntergekühlt. Im 2. Kristaller wird der Mischkristallbrei mit 1000 m³/h umgewälzt. Aus dem 2. Kristaller wird die Mischkristallbrei-Lösung mit 22 m³/h ausgespeist und dem Drehfilter zugeführt. Hier werden ca. 5 to Mischkristalle abfiltriert, gewaschen und getrocknet. Das Filtrat enthalt einen Rest-Bisphenol-A-Gehalt von 14 %. Die Reinheit des abfiltrierten und aufgearbeiteten Bisphenol-A-Endproduktes beträgt 99,90 % p,p-BPA, die Farbzahl der abgeschuppten BPA-Ware betrug 5-10 Hazen. Die Verweilzeit des Mischkristallbreis in den Kristallerkaskaden lag bei 9 h.

### Beispiel 2

45 m³/h einer 28 %igen phenolischen Bisphenol-A-Lösung wurde mit einer Temperatur von 70°C in einen Kristaller eingespeist und im Kristaller auf 54°C abgekühlt. Der entstehende, phenolische BPA-/Phenol-Mischkristallbrei wurde kontinuierlich mit 1200 m³/h umgewälzt. Aus dem Kristaller wurde der Mischkristallbrei, der eine Temperatur von 54°C besitzt, ausgespeist und in einen zweiten Kristaller eingespeist (45 m³/h). Im 2. Kristaller wurde der Mischkristallbrei auf 41°C weiter heruntergekühlt. Im 2. Kristaller wurde der Mischkristallbrei mit 1000 m³/h umgewälzt. Aus dem zweiten Kristaller wurde die Mischkristallbrei-Lösung mit 45 m³/h ausgespeist und dem Drehfilter zugeführt. Hier wurden ca. 10 to Mischkristalle abfiltriert, gewaschen und getrocknet. Das Filtrat enthielt einen Rest-Bisphenol-A-Gehalt von 14 %. Die Reinheit des abfiltrierten und aufgearbeiteten Bisphenol-A-Endproduktes betrug 99,75 % p,p-BPA, die Farbzahl der BPA-Schuppenware Hazen 10. Die Verweilzeit des Mischkristallbreis in der Kristallerkaskade lag bei 4 h.

### Beispiel 3

8 m³/h einer 32 %igen phenolischen Bisphenol-A-Lösung wurden mit einer Temperatur von 70°C in einen Kristaller eingespeist und im Kristaller auf 54°C abgekühlt. Der entstehende, phenolische BPA-/Phenol-Mischkristallbrei wurde kontinuierlich mit 500 m³/h umgewälzt. Aus dem Kristaller wird der Mischkristallbrei, der eine Temperatur von 54°C besitzt, ausgespeist und in einen zweiten Kristaller eingespeist (8 m³/h). Im 2. Kristaller wird der Mischkristallbrei auf 41°C weiter heruntergekühlt. Im 2. Kristaller wird der Mischbrei mit 550 m³/h umgewälzt. Aus dem 2. Kristaller wird der Mischkristallbrei mit 8 m³/h ausgespeist und dem Drehfilter zugeführt. Hier werden ca. 2,0 % Mischkristalle abfiltriert, gewaschen und getrocknet. Die Reinheit des abfiltrierten und aufgearbeiteten Bisphenol-A-Endproduktes beträgt 99,94 % p,p-BPA, die Farbzahl der abgeschuppten Ware betrug 5 Hazen. Die Verweilzeit lag bei ca. 20 h.

### Beispiel 4

10 m³/h einer 29 %igen phenolischen Bisphenol-A-Lösung wurde mit 72°C in einen ersten von 3 Kristallern eingespeist und auf 56°C abgekühlt. Der entstehende phenolische Bisphenol A/Phenol-Mischkristallbrei wurde dabei mit 1000 m³/h umgewalzt. Der 1. Kristaller lief in einen zweiten Kristaller über, der auf 49°C halten wurde. Wiederum erfolgte die Abkühlung durch eine Umwälzmenge von 1000 m³/h. Schließlich wurde im 3. Kristaller auf eine Endtemperatur von 41°C abgekühlt. Die auf diese Weise erzeugten Bisphenol A/Phenol-Mischkristalle wurden auf übliche Werte in einem Drehfilter abgetrennt und zu reinem Bisphenol aufgearbeitet.

Die Reinheit der erzeugten Bisphenols lagen bei 99,92 % und die Farbzahl bei Hazen 5-10. Die Verweilzeit in der Kristallerkaskade lag bei insgesamt 18 h.

### Vergleichsbeispiel 1 (Fahrweise mit einem Kristaller)

Es wird gemäß Beispiel 2 verfahren mit dem Unterschied, daß der 1. Kristaller mit einer Temperatur von 54°C nicht betrieben wird. Alle anderen Verfahrensparameter bleiben gleich. Die Reinheit des abfiltrierten und aufgearbeiteten Bisphenol-A-Endproduktes betrug 99,52 % pp-BPA, die Farbzahl der Schuppenware ist 30. Die Verweilzeit des Mischkristallisates im Kristaller 2 (T = 41°C) lag bei ca. 2 h.

### Vergleichsbeispiel 2 (Fahrweise mit 2 Kristallern, aber beide werden auf 41°C eingestellt)

Es wird gemäß Beispiel 2 verfahren. Alle Verfahrensparameter bleiben gleich, nur die Temperatur von Kristaller 1 wird auf 41°C abgesenkt.

Die Reinheit des abfiltrierten und aufgearbeiteten Bisphenol-A-Endproduktes betrug 99,62 % p,p-BPA, die Farbzahl der Schuppenware ist 25. Die Verweilzeit des Mischkristallisates in beiden Kristallern (T₁ = 41°C, T₂ = 41°C) lag bei 4 h.

## Patentansprüche

1. Verfahren zur Gewinnung von Bisphenol-A mit einem Reinheitsgrad von 99,75 bis >99,94 Gew.-% und einer Farbzahl nach Hazen von 5 bis 10 aus der Reaktionslösung, die bei der Herstellung von Bisphenol-A aus Aceton und Phenol in Gegenwart von gegebenenfalls mit Mercaptoaminen und/oder Thiazolidinen und/oder Thiocarbonsäuren modifizierten sulfonsauren Ionenaustauschern erhalten wird, dadurch gekennzeichnet, daß
1. die Reaktionslösung bei einer Temperatur von 65 bis 75°C auf einen Gehalt an 25 bis 35 Gew.-% p,p-Bisphenol-A in Phenol eingestellt wird und
2. anschließend mit dieser Temperatur in eine n-stufige, seriengeschaltete Kristallerkaskade mit einer Anzahl an Kristallisationsreaktoren n > 1 eingespeist wird und
3. mit einer Verweilzeit des Gemisches pro Kristallisationsreaktor (n) von mindestens drei Stunden und einer Umpumpleistung von mindestens 500 m³/h im jeweiligen Kristallisationsreaktor umgepumpt wird und
4. über die gesamte Kristallisationskaskade ein Temperaturgefalle von 70°C im ersten Kristallisationsreaktor (n = 1) auf 40°C im letzten Kristallisationsreaktor(n) eingestellt wird und
5. p,p-Bisphenol-A-Addukt-Kristallisate mit einem Gehalt an p,p-BPA von ca. 60 % und Phenol von ca. 40 % in den Kristallreaktoren gefällt, dann abfriltriert, Phenol entfernt und p,p-Bisphenol in üblicher Weise gewonnen wird.

## Claims

1. Process for recovering bisphenol A of a purity of 99.75 to > 99.94 wt.% and with a Hazen colour value of 5 to 10 from the reaction solution obtained on production of bisphenol A from acetone and phenol in the presence of sulphonic acid ion exchangers optionally modified with mercaptoamines and/or thiazolidines and/or thiocarboxylic acids, characterised in that
1. the reaction solution is adjusted to a content of p,p-bisphenol A in phenol of 25 to 35 wt.% at a temperature of 65 to 75°C and
2. is then fed at this temperature into an n-stage cascade of crystallisers connected in series with a number of crystallisation reactors of n > 1 and
3. is circulated in each crystallisation reactor at a circulation rate of at least 500 m³/h with a residence time of the mixture in each crystallisation reactor (n) of at least three hours and
4. a temperature gradient from 70°C in the first crystallisation reactor (n = 1) to 40°C in the final crystallisation reactor (n) is arranged over the entire crystallisation cascade and
5. p,p-bisphenol A adduct crystallisate with a p,p-BPA content of approximately 60% and a phenol content of approximately 40% is introduced into the crystal reactors, then filtered out, phenol removed and p,p-bisphenol recovered in a customary manner.

## Revendications

1. Procédé pour préparer du bisphénol-A à un degré de pureté de 99,75 à plus de 99,94 % en poids et à un indice de couleur Hazen de 5 à 10 à partir de la solution de réaction obtenue à la préparation du bisphénol-A à partir de l'acétone et du phénol en présence d'échangeurs d'ions acides sulfoniques éventuellement modifiés par des mercaptoamines et/ou des thazolidines et/ou des acides thiocarboxyliques, caractérisé en ce que
1. on règle la solution de réaction à une teneur de 25 à 35 % en poids de p,p-bisphénol-A dans le phénol à une température de 65 à 75°C et
2. on l'envoie à la même température dans une série de cristallisoires à n étages comportant un nombre de réacteurs de cristallisation n < 1, et
3. on soumet le mélange à circulation par pompe au débit d'au moins 500 m³/h dans chaque réacteur de cristallisation avec une durée de passage du mélange d'au moins 3 h par réacteur de cristallisation (n) et
4. on établit sur toute la série de cristallisoirs une diminution de température de 70°C au premier réacteur de cristallisation (n = 1) à 40°C dans le dernier réacteur de cristlalisation (n) et
5. on précipite dans les réacteurs de cristallisation des cristaux de l'adduct p,p-bisphénol-A/phénol à une teneur en p,p-BPA d'environ 60 % et une teneur en phénol d'environ 40 %, on les filtre, on sépare le phénol et on isole le p,p-bisphénol de la manière habituelle.
